# EUROPEAN PATENT SPECIFICATION

(11) **EP 0 475 364 B1**
(45) Date of publication and mention of the grant of the patent: **23.12.1998**
(21) Application number: 91115302.1
(22) Date of filing: 10.09.1991
(51) Int. Cl.: C07D 251/52

(54) **Diamino-s-triazinone derivatives**
Diamino-s-Triazinonderivate
Dérivés de la diamino-s-triazinone

(30) Priority: 11.09.1990 IT 2142290
(43) Date of publication of application: 18.03.1992
(73) Proprietor: MINISTERO DELL' UNIVERSITA' E DELLA RICERCA SCIENTIFICA E TECNOLOGICA, 00196 Roma (IT)
(72) Inventor: Cipolli, Roberto, Dr., I-28100 Novara (IT); Nucida, Gilberto, I-20098 San Giuliano Milanese, Milan (IT); Masarati, Enrico, Dr., I-29010 Castelnuovo Valtidone, Piacenza (IT); Oriani, Roberto, I-20137 Milan (IT); Pirozzi, Mario, Dr., I-20097 San Donato Milanese, Milan (IT)
(74) Representative: Barz, Peter, Dr.

(56) References cited:
- JP-A-61 126 091
- US-A- 4 542 170
- CHEMICAL ABSTRACTS, vol. 110, no. 19, May 8, 1989, Columbus, Ohio, USA INOUE, YOSHINORI et al. "Synthesis of 4-hydroxy-2- -phenylazo-1,3,5-triazines by oxidative coupling of 2-hydra-zino-4-hydroxy-1,3,5-triazineswith N,N-dialkylanilines." page 772, column 2, abstract-no. 173 192g
- CHEMICAL ABSTRACTS, vol. 93, no. 22, December 1, 1980, Columbus, Ohio, USA MITSUBISHI PAPER MILLS, LTD. "Silver halide photographic material backcoating." page 590, column 2, abstract-no. 213 311j
- CHEMICAL ABSTRACTS, vol. 87, no. 5, August 1, 1987, Columbus, Ohio, USA TSUJIKAWA, TERUAKI et al. "Photochemical reactions of 2-benzylidenehydrazino- pyrimidines." page 513, column 2, abstract-no. 39 407j
- CHEMICAL ABSTRACTS, vol. 74, no. 25, June 21, 1971, Columbus, Ohio, USA NAKANISHI, MICHIO et al. "s-Triazine derivatives." page 601, column 2, abstract-no. 141 881c
- CHEMICAL ABSTRACTS, vol. 69, no. 21, November 18, 1968, Columbus, Ohio, USA BREDERECK, HELLMUT et al. "Syntheses of heterocycles. XII. Substitued s-triazines. Synthesis and reactions of 6-hydroxy-2,4-bis(nitramino)- -s-triazine." page 8134, column 2, abstract-no. 86 965c
- CHEMICAL ABSTRACTS, vol. 62, no. 9, April 26, 1965, Columbus, Ohio, USA R. ANGELUCCI et al. "Some aspects of the meta- bolism of triazine derivativesactive in experimentally in- duced virus infections." column 11003, abstract-no. 11 003h

## Description

The present invention relates to derivatives of 2,4-diamino-6-hydroxy-1,3,5-triazine (ameline) which compounds are capable of endowing thermoplastic polymers and polymers having elastomeric properties, especially olefin polymers and copolymers, with self-extinguishing properties.

In particular, the present invention provides compounds of general formula (I): 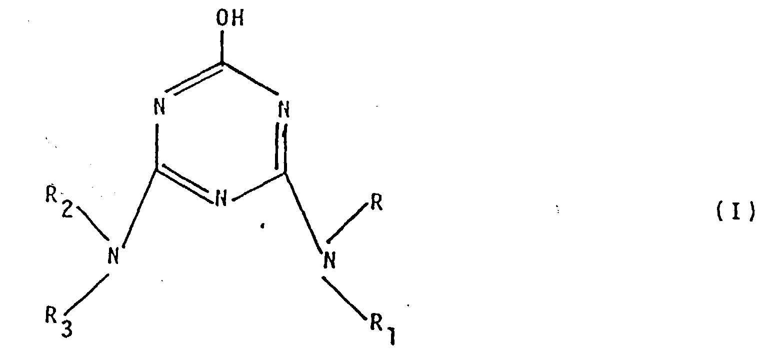 wherein:
R is hydrogen;
and at least one of the groups R₁, R₂ and R₃ is selected from ⁅CₙH₂ₙ⁆O-R₄; and 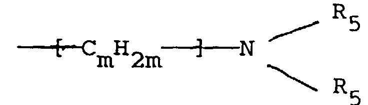
wherein:
n = integer of from 2 to 8, preferably from 2 to 4;
m = integer of from 2 to 6;
R₄ = H; (C₁-C₈), and preferably (C₁-C₄)-alkyl; (C₂-C₆)-alkenyl;
   ⁅CₚH₂ₚ⁆O-R₆, p being an integer of from 1 to 4 and R₆ being H or (C₁-C₄)-alkyl; (C₆-C₁₂)-cycloalkyl; or (C₆-C₁₂)-alkylcycloalkyl;
the groups R₅, the same or different from each other, represent H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₅)₂ is replaced by an N-heterocyclic radical which is linked to the alkyl chain through the nitrogen atom and is selected from aziridyl.; pyrrolidyl; piperidyl; morpholyl; thiomorpholyl; piperazyl; 4-methylpiperazyl and 4-ethylpiperazyl;
the remaining groups R₁ to R₃, the same or different from each other, having the above meanings or being selected from H; (C₁-C₁₈)-alkyl; (C₂-C₈)-alkenyl; (C₆-C₁₆)-cycloalkyl and (C₆-C₁₆)-alkylcycloalkyl, optionally substituted with a hydroxy or (C₁-C₄)-hydroxyalkyl group;
or in general formula (I) at least one of the moieties -NRR₁ and -NR₂R₃ is replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
provided that (a) when one of R₂ and R₃ is CH₂CH₂OH and the other one is CH₂CH₂OH or (C₁₋₁₂)-alkyl, -NRR₁ cannot represent -NHR* wherein R* is hydrogen or (C₁₋₁₀)-alkyl, or be replaced by aziridinyl, pyrrolidyl, piperidyl and 4-methylpiperazyl or 4-ethylpiperazyl; (b) when R₁ is hydrogen, -NR₂R₃ cannot represent a morpholino group and (c) the moieties -NRR₁ and -NR₂R₃ cannot represent two morpholino groups or two piperidino groups.

The above provisos are based on the disclosures of US-A- 3 806 475; Heterocycles, 1977, 6(4), 423-9; Chemische Berichte, 1968, 101(9), 3062-9; and Brit. J. Pharmacol. 1965, 24(1); 274-81 wherein corresponding ameline derivatives are disclosed, e.g., as catalysts for carbodiimide formation in polyisocyanate-based cellular foams and as antiviral agents.

Specific examples of groups R₁ to R₃ in general formula (I) are methyl; ethyl; propyl, isopropyl; n-butyl; isobutyl; tert-butyl; n-pentyl; isopentyl; n-hexyl; tert-hexyl; octyl; tert-octyl; decyl; dodecyl; octadecyl; ethenyl; propenyl; butenyl; isobutenyl; hexenyl; octenyl; cyclohexyl; propylcyclohexyl; butylcyclohexyl; decylcyclohexyl; hydroxycyclohexyl; hydroxyethylcyclohexyl; 2-hydroxyethyl; 2-hydroxypropyl; 3-hydroxypropyl; 3-hydroxybutyl; 4-hydroxybutyl; 3-hydroxypentyl; 5-hydroxypentyl; 6-hydroxyhexyl; 3-hydroxy-2,5-dimethylhexyl; 7-hydroxyheptyl; 7-hydroxyoctyl; 2-methoxyethyl; 2-methoxypropyl; 3-methoxypropyl; 4-methoxybutyl; 6-methoxyhexyl; 7-methoxyheptyl; 7-methoxyoctyl; 2-ethoxyethyl; 3-ethoxypropyl; 4-ethoxybutyl; 3-propoxypropyl; 3-butoxypropyl; 4-butoxybutyl; 4-isobutoxybutyl; 5-propoxypentyl; 2-cyclohexyloxyethyl; 2-ethenyloxyethyl; 2-(N,N-dimethylamino)ethyl; 3-(N,N-dimethylamino)propyl; 4-(N,N-dimethylamino)butyl; 5-(N,N-dimethylamino)pentyl; 4-(N,N-diethylamino)butyl; 5-(N,N-diethylamino)pentyl; 5-(N,N-diisopropylamino)pentyl; 3-(N-ethylamino) -propyl; 4-(N-methylamino)butyl; 4-(N,N-dipropylamino)butyl; 2-(N,N-diisopropylamino)ethyl; 6-(N-hexenylamino)hexyl; 2-(N-ethenylamino)ethyl; 2- (N-cyclohexylamino)ethyl; 2- (N-2-hydroxyethylamino)ethyl; 2-(2-hydroxyethoxy)ethyl; 2-(2-methoxyethoxy)ethyl; 6-(N-propylamino)hexyl.

Specific compounds of general formula (I) are given in the examples following the present description.

The compounds of general formula (I) can be prepared by hydrolysis of intermediates of general formula (II): 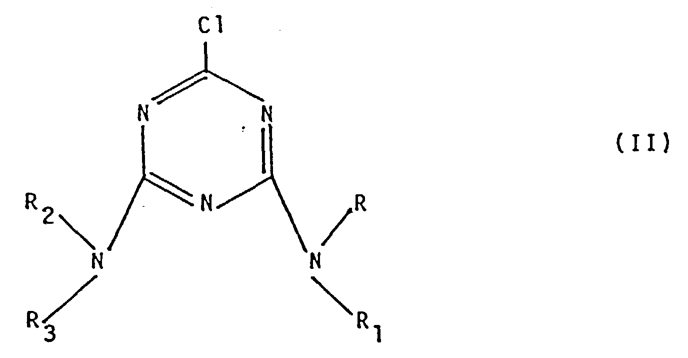 (wherein R, R₁, R₂ and R₃ have the previously defined meanings) with an acid (such as hydrochloric acid; hydrobromic acid; sulfuric acid; phosphoric acid) at temperatures of from about 60 to about 100°C, or with a base (such as sodium hydroxide; potassium hydroxide) at temperatures of from about 100 to about 180°C.

The product formed can easily be separated from the reaction mass by filtration.

Intermediates of general formula (II) can easily be synthesized by reacting a cyanuric acid halide, for instance the chloride, at a temperature of from about 0 to about 10°C and in a suitable solvent (such as acetone, water, methylene chloride, etc.), with an amine of general formula (III): 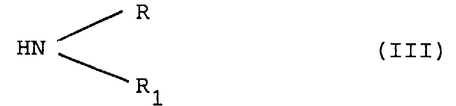 wherein R and R₁ have the previously defined meanings, in the presence or absence (depending on the molar ratio used) of an acidity acceptor (such as NaOH, NaHCO₃, Na₂CO₃ and triethylamine), thereby obtaining the intermediate of general formula (IV):

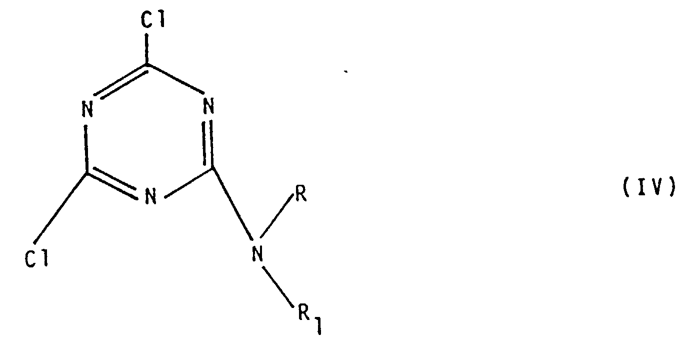

This intermediate, either separated or not, is subsequently allowed to react under conditions analogous to the preceding ones, but working at a higher temperature, for instance of from about 10 to about 50°C, with an amine of the general formula (V): 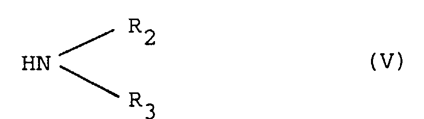 wherein R₂ and R₃ have the previously defined meanings, thereby obtaining the intermediate (II).

If intermediates of general formula (II) wherein -NRR₁ = -NR₂R₃ are desired, the cyanuric acid halide is allowed to react with two mols (in the presence of an acidity acceptor) or with four mols (in the absence of an acidity acceptor) of an amine of general formula (III) under working conditions analogous to those described previously.

An alternative method for obtaining derivatives of general formula (I) is to hydrolyse, either with an acid (working at from about 80 to about 150°C) or with a base (working at about 100 to about 180°C) and using the same reagents stated above for the hydrolysis of intermediates of general formula (II), compounds of general formula (VI): 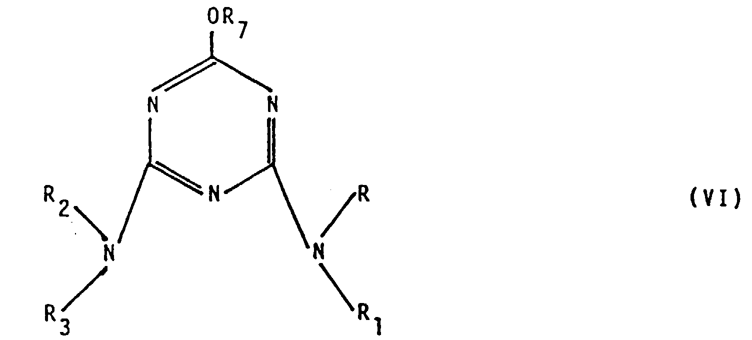 wherein R, R₁, R₂ and R₃ have the previously defined meanings and R₇ is preferably (C₁-C₄)-alkyl.

Compounds of general formula (VI) can be prepared, e.g., by condensation of the intermediate of general formula (II) with a reagent of general formula (VII):

R₇-OH (VII)

wherein R₇ has the previously defined meaning; in a suitable solvent (such as toluene, xylene, ortho-dichlorobenzene) and/or in an excess of the compound (VII) if the latter can act as solvent (such as in the case of methanol, ethanol ) in the presence of a base (such as sodium hydroxide, potassium hydroxide, sodium metal.) at temperatures of from about 60 to about 150°C.

Generally products of general formula (I) showing good properties are obtained in the form of a white crystalline powder which can be employed in self-extinguishing polymeric compositions without any further purification.

### EXAMPLE 1

Into a 2 1-reactor equipped with stirrer, thermometer, dropping funnel, condenser and cooling bath, there are placed 184.5 g of cyanuric acid chloride and 1300 ml of methylene chloride.

While cooling externally, 87.2 g of morpholine and 40 g of sodium hydroxide dissolved in 150 g of water are added simultaneously within 3 hours, keeping the pH between 5 and 7 and the temperature between 0 and 3°C.

The mixture is kept at 0 to 3°C for a further 3 hours and thereafter the aqueous phase is separated.

Upon distillation of the methylene chloride, 230 g of the intermediate of formula (VIII): 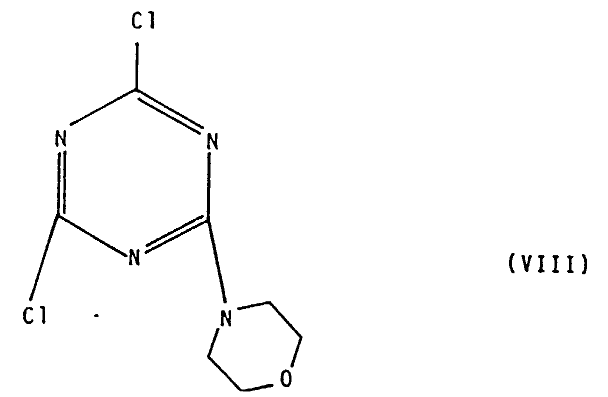 are obtained in the form of a white crystalline powder having a melting point (m.p.) of 155-157°C and a chlorine content of 30.12% (theor. = 30.21%).

In a 1 l-reactor equipped with stirrer, thermometer, feeding funnel, cooler and heating bath, there are introduced 300 ml of water, 30.5 g of 2-hydroxyethylamine and, under stirring, 117.5 g of the intermediate (VIII).

The temperature is gradually raised to 40°C; after 30 minutes the mixture is heated to 45°C and is kept at this temperature for about 3 hours.

Then the temperature is raised to 50°C and within 3 hours a solution of 20 g of sodium hydroxide in 100 ml of water is added.

The resulting mixture is kept at 50°C for a further 2 hours and thereafter is heated to 70°C and is allowed to further react at this temperature for 30 minutes.

After cooling to room temperature, the product formed is filtered off and washed on the filter with water.

After drying of the cake in an oven at 100°C, 120.3 g of the intermediate of formula (IX): 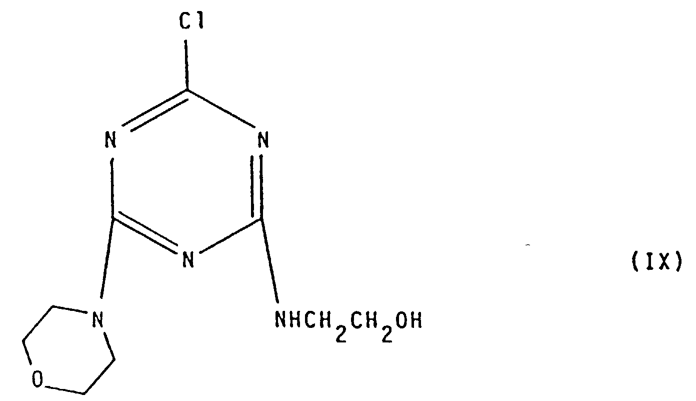 are obtained in the form of a white crystalline powder the m.p. of which is 172-173°C and the chlorine content of which is 13.51% (theor.: 13.68%).

The structure of the intermediates (VIII) and (IX) was further confirmed by IR spectroscopic analysis.

The same 1 l reactor is charged with 500 ml of water, 103.8 g of the intermediate (IX) and 79 g of a 37% by weight solution of hydrochloric acid.

The mass is heated to 90°C and is kept at this temperature for 3 hours. Then the solution is cooled to 50°C and is neutralized by adding 48 g of sodium hydroxide dissolved in 80 ml of water.

The resulting mixture is cooled to 5°C and the product formed is filtered and washed on the filter with cold water.

Upon drying the filter cake in an oven at 100°C, 84.5 g of the product of formula 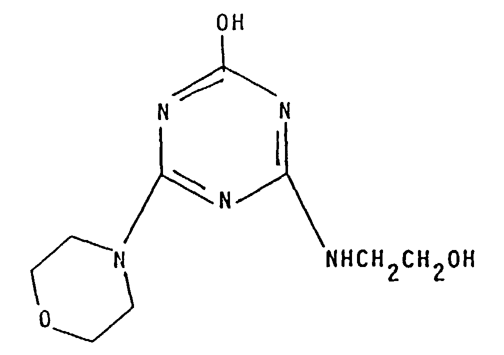 are obtained in the form of a white crystalline powder; m.p. = 251-253°C.

### EXAMPLE 2 (reference example)

Into a 2 l-reactor, equipped as in example 1, 800 ml of methanol, 100 ml of water and 151.2 g of sodium bicarbonate are introduced.

The mixture is cooled to 10°C and then 166 g of cyanuric acid chloride are added.

The temperature is allowed to rise up to 30°C and is kept at this value for about 1 hour, until completion of the carbon dioxide evolution.

The heat of reaction itself is sufficient for maintaining the desired temperature.

Then the reaction mixture is cooled to 5°C and thereafter 800 ml of cold water are added. The product formed is filtered off and washed on the filter with cold water.

Upon drying the cake in an oven under vacuum at 60°C, 123.8 g of the intermediate of formula (X): 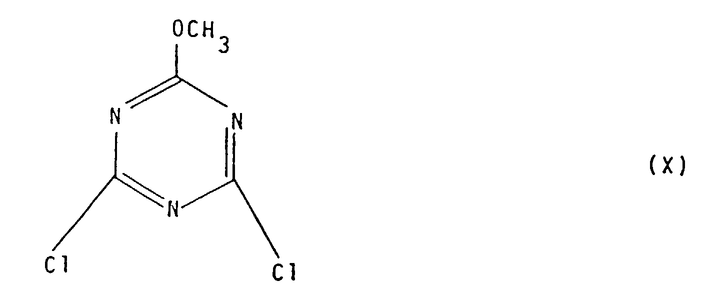 are obtained in the form of a white crystalline powder; m.p. = 90-92°C; chlorine content 39.27% (theor. 39.44%).

In a 1 l-reactor, equipped as in example 1, 400 ml of water and 108 g of the intermediate (X) are introduced.

While cooling externally to 0-5°C, 100 g of a 30% by weight aqueous ammonia solution are added within about 1 hour while keeping the temperature at 0 to 5°C. Then the temperature is allowed to rise spontaneously to room temperature and this value is maintained for 2 hours.

The resulting mixture is cooled to 10°C and the product formed is filtered off and washed with cold water. By drying the cake in an oven at 100°C, 82.3 g of the intermediate of formula (XI): 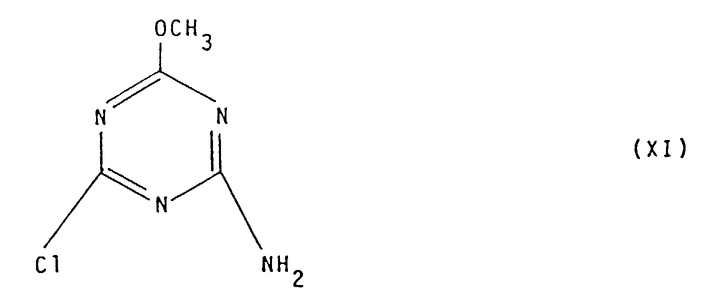 are obtained in the form of a white crystalline powder; m.p. >300°C; chlorine content 21.92% (theor.: 22.12%).

The structure of intermediates (X) and (XI) was further confirmed by NMR analysis.

In the same 1 l-reactor, but provided with a heating bath, 300 ml of toluene, 80.2 g of the intermediate (XI) and 90 g of morpholine are introduced.

The mixture is heated to 60 to 65°C and is kept at this temperature for 2 hours; thereafter the mixture is heated to reflux and is kept in this condition for 1 hour.

After having allowed the mixture to cool to room temperature, the product formed is separated by filtration.

The cake is thoroughly washed with water and, after drying, 90.3 g of the product of formula (XII): 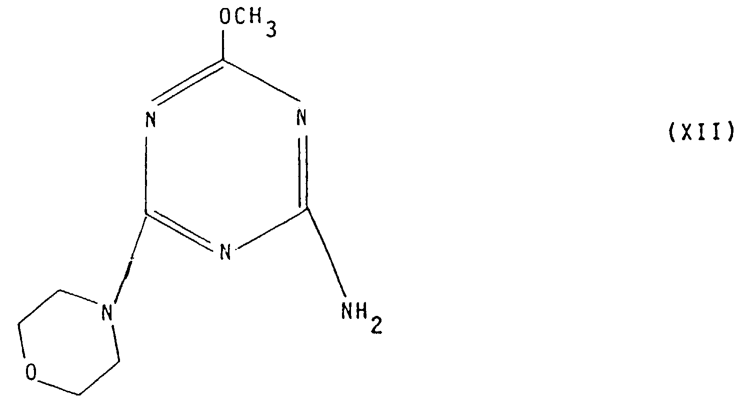 are obtained in the form of a white crystalline powder; m.p. = 182-184°C.

In the same 1 l-reactor 400 ml of water, 63.3 g of intermediate (XII) and 59.1 g of a 37% by weight hydrochloric acid solution are placed.

The mixture is heated to boiling and kept under reflux for 2 hours.

After cooling to 80°C, 24 g of sodium hydroxide dissolved in 100 ml of water are added.

The resulting mixture is allowed to cool to room temperature, then the product formed is filtered off and washed on the filter with water.

Upon drying the cake in an oven at 100°C, 54.7 g of the product of formula: 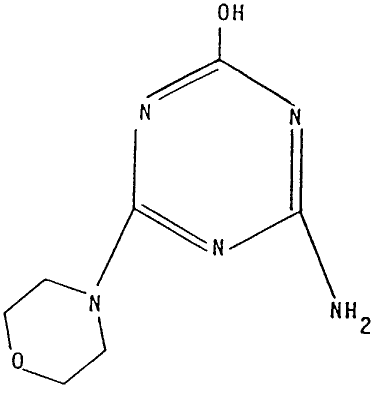 are obtained in the form of a white crystalline powder; m.p. >300°C.

### EXAMPLE 3

600 ml of water and 184.5 g of cyanuric acid chloride are placed in a 2 l reactor equipped as in example 1.

While externally cooling to 2°C, 122.5 g of 2-hydroxyethylamine in 100 ml of water are added within 2 hours; during the addition the temperature is allowed to gradually rise up to 5 to 7°C.

Then the temperature is raised to 20°C and is kept at this value for 1 hour; thereafter the whole mixture is heated to 35 to 40°C and 80 g of sodium hydroxide (dissolved in 200 ml of water) are added within about 3 hours.

The reaction mass is heated to 60°C and is kept at this temperature for 2 hours, whereafter it is cooled to room temperature and the product formed thereby is filtered off and washed on the filter with water.

By drying the cake in an oven at 100°C, 203.1 g of the intermediate of formula (XIII): 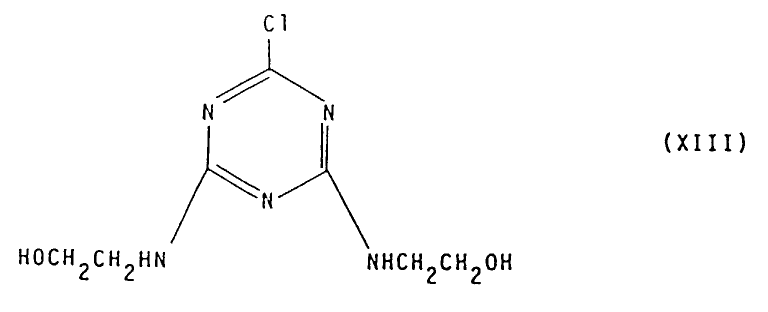 are obtained in the form of a white crystalline powder; m.p. = 188-190°C; chlorine content 15.33% (theor. 15.20%).

The structure of the intermediate (XIII) was further confirmed by IR spectroscopic analysis.

400 ml of water, 13 g of sodium hydroxide and 70.1 g of the intermediate (XIII) are placed in a 1 l stainless steel reactor equipped as in example 1.

The mixture is then heated to 150°C and is kept at this temperature for about 10 hours.

Thereafter the reaction mass is cooled to room temperature and the product formed is filtered and washed on the filter with water.

By drying the cake in an oven at 100°C, 57.7 g of the product of formula 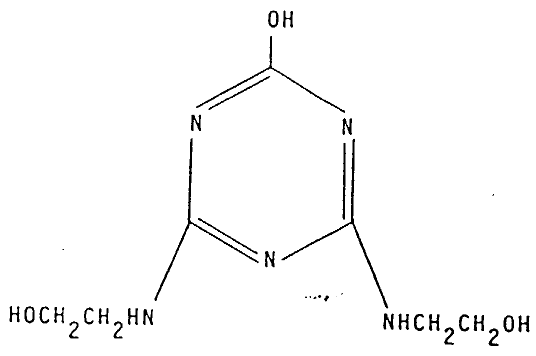 are obtained in the form of a white crystalline powder; m.p. >300°C.

### EXAMPLE 4

1000 ml of methylene chloride, 129.1 g of cyanuric acid chloride, 51.2 g of tert-butylamine and 28 g of sodium hydroxide dissolved in 100 ml of water are placed in the 2 l-reactor described in example 1.

By working as described in example 1, after having distilled off the solvent, 148 g of the intermediate of formula (XIV): 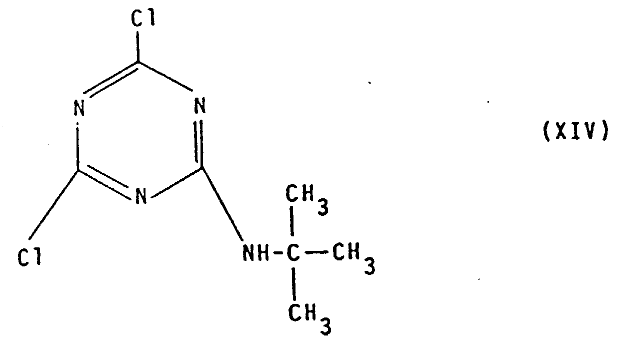 are obtained in the form of a white crystalline powder; m.p. = 129-130°C; chlorine content 31.87% (theor. 32.13%).

500 ml of chloroform, 110.5 g of the intermediate (XIV) and 30.5 g of 2-hydroxyethylamine, dissolved in 80 ml of water, are placed in a 1 l-reactor equipped as in the preceding examples.

The mixture is heated to boiling and kept under reflux for 3 hours; thereafter, a solution of 20 g of sodium hydroxide in 70 ml of water is added within 2 hours.

The mixture is kept under reflux for a further hour and then is cooled to room temperature by separating the organic phase.

The solvent is distilled off and the distillation residue is treated with 500 ml of water in the same 1 l-reactor.

The product is heated to 50 to 60°C until a good dispersion is obtained and thereafter the mixture is cooled to room temperature and the product formed is separated by filtration.

The filter cake is washed with water and dried in an oven at 80°C.

106.7 g of the intermediate of formula (XV): 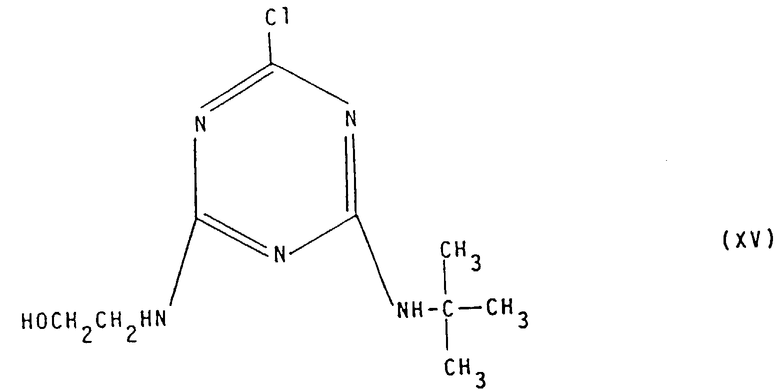 are obtained in the form of a white crystalline powder; m.p. = 134-135°C; chlorine content 14.32% (theor. 14.46%).

The structure of intermediates (XIV) and (XV) was further confirmed by NMR analysis.

500 ml of water, 98.2 g of the intermediate (XV) and 20.5 g of 96% by weight sulfuric acid are placed in the same 1 l-reactor.

The reaction mass is heated to 85°C and is kept at this temperature for 2 hours.

Thereafter, 32 g of sodium hydroxide, dissolved in 100 ml of water, are added within 30 minutes.

The resulting mixture is kept at 85°C for a further 30 minutes and thereafter is cooled to room temperature; the product formed is filtered and washed on the filter.

Upon drying the cake in an oven at 100°C, 83.2 g of the product of formula 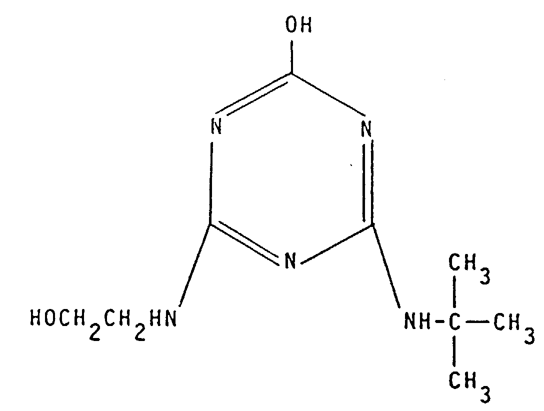 are obtained in the form of a white crystalline powder; m.p. >300°C.

### EXAMPLE 5

600 ml of water and 184.5 g of cyanuric acid chloride are introduced in a 2 l-reactor equipped as described in example 1.

While cooling from the outside to 0 to 2°C, 75 g of 2-methoxyethylamine are added within 1.5 hours.

Subsequently, 40 g of sodium hydroxide, dissolved in 250 ml of water, are introduced within 2 hours, always maintaining the temperature at 0-2°C.

The mass is kept under stirring for a further hour at the same temperature, then the product formed is separated by filtration and washed on the filter with water.

By drying in an oven under vacuum at 60°C, 178.9 g of the intermediate of formula (XVI): 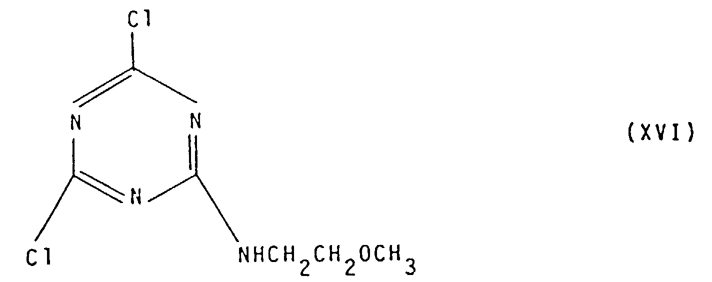 are obtained in the form of a white crystalline powder; m.p. = 73-75°C; chlorine content 31.68% (theor. 31.84%).

In a 1 l-reactor, equipped as in example 1, there are introduced 85 g of a 30% by weight aqueous ammonia solution, 250 ml of water and 111.5 g of intermediate (XVI).

The mixture is first heated to 40°C, keeping this temperature for 4 hours, then to 55°C for 2 hours. Thereafter the reaction mass is cooled to 10°C and the product formed is filtered and washed on the filter with water.

By drying the cake in an oven at 100°C, 98 g of the intermediate of formula (XVII): 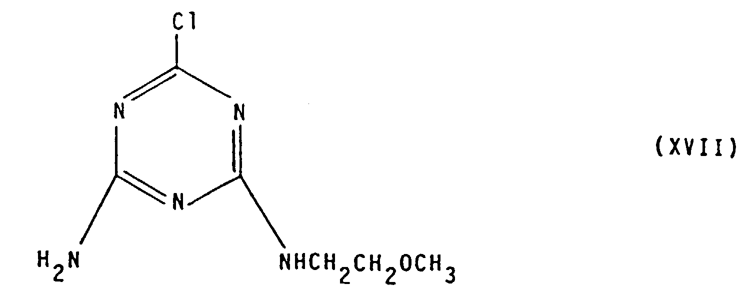 are obtained in the form of a white crystalline powder; m.p. = 195-197°C; chlorine content 17.21% (theor. 17.44%).

The structure of intermediates (XVI) and (XVII) was further confirmed by IR spectroscopic analysis.

400 ml of water, 81.4 g of the intermediate (XVII) and 42.3 g of a 37% by weight hydrochloric acid are placed in the same 1 l-reactor.

The mixture is heated to 80°C and is kept at this temperature for 2 hours.

Thereafter, 44.2 g of sodium carbonate, dissolved in 200 ml of water, are added, always at 80°C.

The reaction mass is then cooled to room temperature and the product formed is filtered and washed on the filter with water.

By drying the cake in an oven at 100°C, 68.1 g of the product of formula 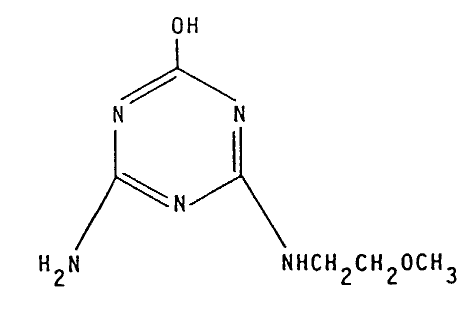 are obtained in the form of a white crystalline powder having a melting point of >300°C.

### EXAMPLES 6 TO 19

By working under conditions analogous to those described in examples 1 to 5 products of general formula (I) reported in Table 1 are prepared.

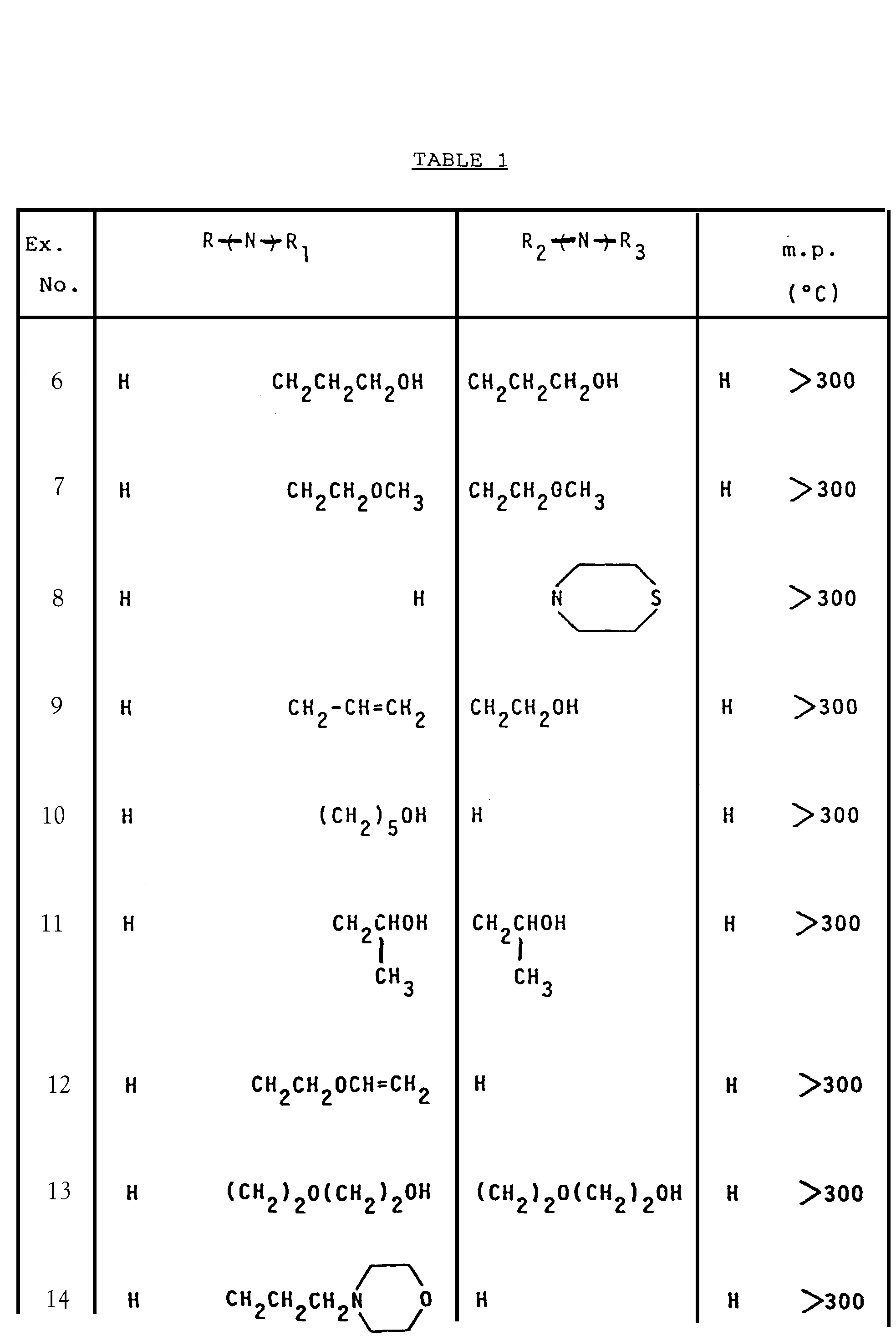

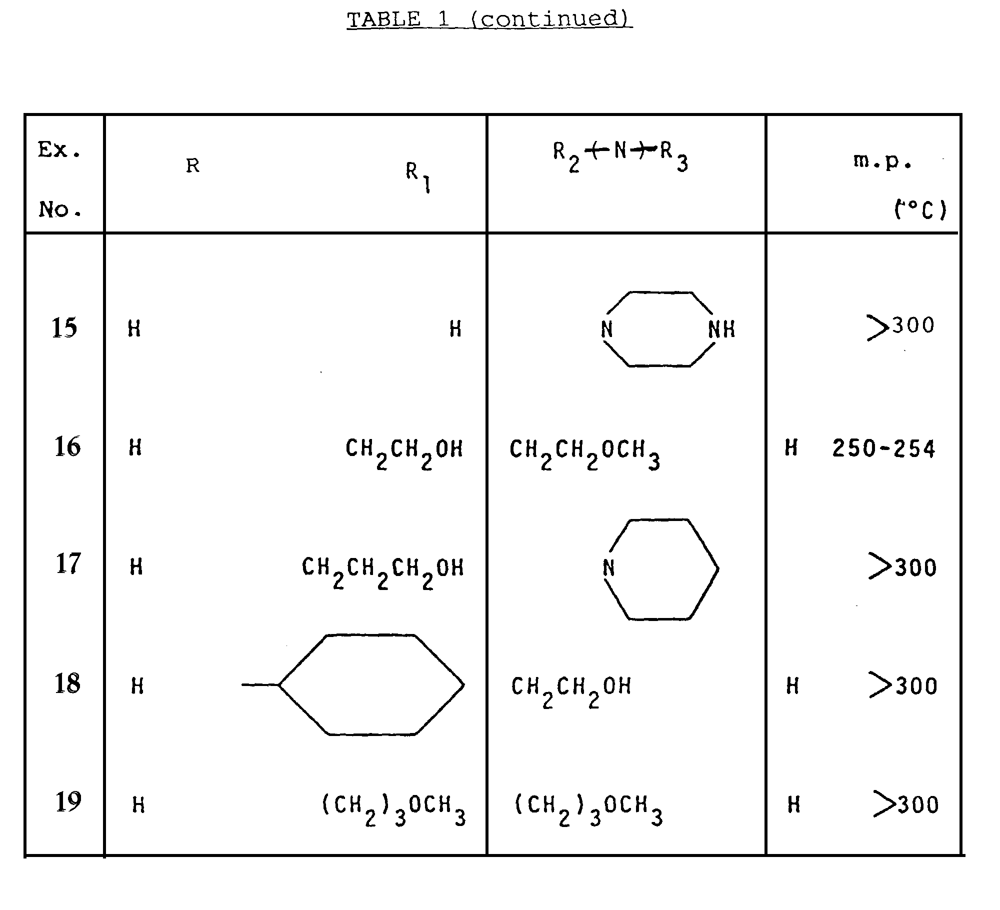

### EXAMPLE 20

75 g of isotactic polypropylene in flake form and having a Melt Flow Index of 12 and a fraction insoluble in boiling n-heptane equal to 96%, 12 g of the product of example 3, 12 g of ammonium polyphosphate (Exolit® 422 by Hoechst), 0.67 g of dilauryl thiopropionate and 0.33 g of pentaerythritol tetra[3-(3,5-di-tert-butyl-4-hydroxyphenyl)propionate] are mixed and molded in a MOORE platen press, working for 7 minutes at a pressure of 40 kg/cm².

Specimens are.obtained in the form of small plates (thickness about 3 mm) on which the self-extinguishing level is determined by measuring the oxygen index (L.O.I. according to ASTM D-2863/77) in a STANTON REDCROFT apparatus and by applying the "Vertical Burning Test" which allows to classify the material according to three ratings, i.e., V-0, V-1 and V-2, according to the Standard UL 94, issued by "Underwriters Laboratories" USA.

The following results are obtained:
- L.O.I. =: 32.9
- UL 94 =: class V-0

## Claims

1. Ameline derivatives of general formula (I): 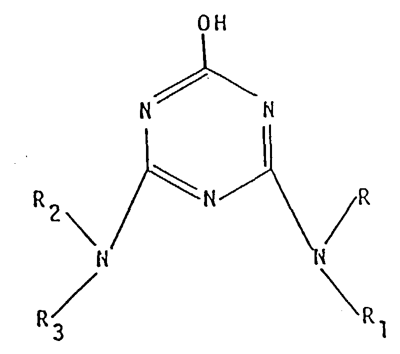 wherein:
R is hydrogen;
and at least one of the groups R₁, R₂ and R₃ is selected from ⁅CₙH₂ₙ⁆O-R₄; and 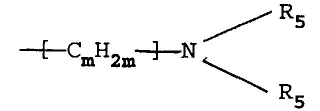
wherein:
n = integer of from 2 to 8;
m = integer of from 2 to 6;
R₄ = H; (C₁-C₈)-alkyl; (C₂-C₆)-alkenyl;
⁅CₚH₂ₚ⁆O-R₆, p being an integer of from 1 to 4 and R₆ being H or (C₁-C₄)-alkyl; (C₆-C₁₂)-cycloalkyl; or (C₆-C₁₂)-alkylcycloalkyl;
the groups R₅, the same or different from each other, represent H; (C₁-C₈)-alkyl; (C₂-C₆)-alkenyl; (C₆-C₁₂)-cycloalkyl; (C₆-C₁₂)-alkylcycloalkyl; and (C₁-C₄)-hydroxyalkyl; or the moiety -N(R₅)₂ is replaced by an N-heterocyclic radical which is linked to the alkyl chain through the nitrogen atom and is selected from aziridyl; pyrrolidyl; piperidyl; morpholyl; thiomorpholyl; piperazyl; 4-methylpiperazyl and 4-ethylpiperazyl;
the remaining groups R₁ to R₃, the same or different from each other, having the above meanings or being selected from H; (C₁-C₁₈)-alkyl; (C₂-C₈)-alkenyl; (C₆-C₁₆)-cycloalkyl and (C₆-C₁₆)-alkylcycloalkyl, optionally substituted with a hydroxy or (C₁-C₄)-hydroxyalkyl group;
or in general formula (I) at least one of the moieties -NRR₁ and -NR₂R₃ is replaced by an N-heterocyclic radical which is linked to the triazine ring through the nitrogen atom and is selected from aziridyl; pyrrolidyl; piperidyl; morpholyl; thiomorpholyl; piperazyl; 4-methylpiperazyl; 4-ethylpiperazyl; 2-methylpiperazyl; 2,5-dimethylpiperazyl; 2,3,5,6-tetramethylpiperazyl; 2,2,5,5-tetramethylpiperazyl; 2-ethylpiperazyl; and 2,5-diethylpiperazyl;
provided that (a) when one of R₂ and R₃ is CH₂CH₂OH and the other one is CH₂CH₂OH or (C₁₋₁₂)-alkyl, -NRR₁ cannot represent -NHR* wherein R* is hydrogen or (C₁₋₁₀)-alkyl, or be replaced by aziridinyl, pyrrolidyl, piperidyl and 4-methylpiperazyl or 4-ethylpiperazyl; (b) when R₁ is hydrogen, -NR₂R₃ cannot represent a morpholino group and (c) the moieties -NRR₁ and -NR₂R₃ cannot represent two morpholino groups or two piperidino groups.

2. Process for the preparation of compounds of general formula (I) according to claim 1, wherein said compounds are obtained by the hydrolysis of intermediates of general formula (II): 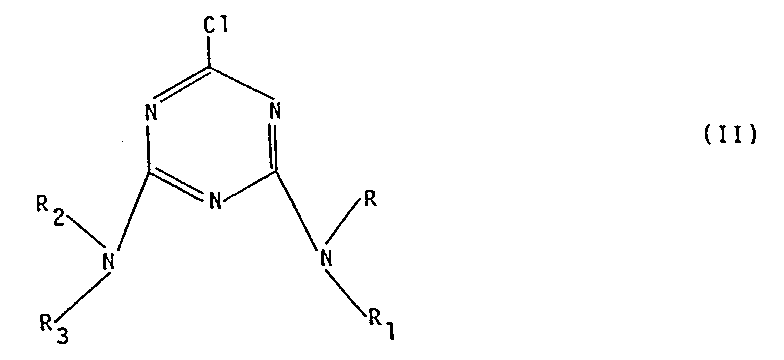 wherein R, R₁, R₂ and R₃ have the meanings given in claim 1.

3. Process according to claim 2, wherein the hydrolysis reaction is carried out in the presence of an acid at temperatures of from about 60 to about 100°C or in the presence of a base at temperatures of from about 100 to about 180°C.

4. Process for the preparation of compounds of general formula (I) according to claim 1, wherein said compounds are obtained by hydrolysis of intermediates of general formula (VI): 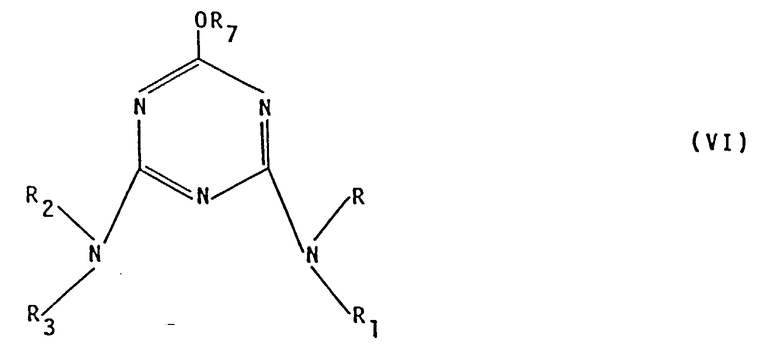 wherein R, R₁, R₂ and R₃ have the meanings given in claim 1 and R₇ is a (C₁-C₄)-alkyl group.

5. Process according to claim 4, wherein the hydrolysis reaction is carried out in the presence of an acid at temperatures from about 80 to about 150°C, or in the presence of a base at temperatures of from about 100 to about 180°C.

## Patentansprüche

1. Amelin-Derivate der allgemeinen Formel (I): 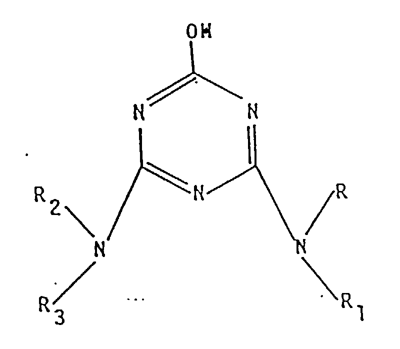 worin:
R Wasserstoff ist;
und mindestens eine der Gruppen R₁, R₂ und R₃ aus ⁅CₙH₂ₙ⁆O-R₄ und 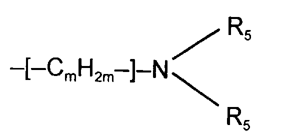
ausgewählt ist, worin:
n= eine ganze Zahl von 2 bis 8;
m= eine ganze Zahl von 2 bis 6;
R₄ = H; (C₁-C₈)-Alkyl; (C₂-C₆)-Alkenyl; ⁅CₚH₂ₚ⁆O-R₆, wobei p eine ganze Zahl von 1 bis 4 und R₆ H oder (C₁-C₄)-Alkyl ist; (C₆-C₁₂)-Cycloalkyl oder (C₆-C₁₂)-Alkylcycloalkyl;
die Gruppen R₅, die gleich oder voneinander verschieden sind, H; (C₁-C₈)-Alkyl; (C₂-C₆)-Alkenyl; (C₆-C₁₂)-Cycloalkyl; (C₆-C₁₂)-Alkylcycloalkyl und (C₁-C₄)-Hydroxyalkyl sind; oder die Einheit -N(R₅)₂ ist durch einen N-heterocyclischen Rest ersetzt, der über das Stickstoffatom mit der Alkylkette verknüpft ist und der aus Aziridyl; Pyrrolidyl; Piperidyl; Morpholyl; Thiomorpholyl; Piperazyl; 4-Methylpiperazyl und 4-Ethylpiperazyl ausgewählt ist;
die übrigen Gruppen R₁ bis R₃, die gleich oder voneinander verschieden sind, die vorstehenden Bedeutungen besitzen oder aus H; (C₁-C₁₈)-Alkyl; (C₂-C₈)-Alkenyl; (C₆-C₁₆)-Cycloalkyl und (C₆-C₁₆)-Alkylcycloalkyl, die gegebenenfalls mit einer Hydroxy- oder (C₁-C₄)-Hydroxyalkylgruppe substituiert sind, ausgewählt sind;
oder mindestens eine der Einheiten -NRR₁ und -NR₂R₃ in der allgemeinen Formel (I) durch einen N-heterocyclischen Rest ersetzt ist, der über das Stickstoffatom mit dem Triazinring verknüpft ist und der aus Aziridyl; Pyrrolidyl; Piperidyl; Morpholyl; Thiomorpholyl; Piperazyl; 4-Methylpiperazyl; 4-Ethylpiperazyl; 2-Methylpiperazyl; 2,5-Dimethylpiperazyl; 2,3,5,6-Tetramethylpiperazyl; 2,2,5,5-Tetramethylpiperazyl; 2-Ethylpiperazyl und 2,5-Diethylpiperazyl ausgewählt ist;
mit der Maßgabe, daß (a) wenn eines von R₂ und R₃ CH₂CH₂OH und das andere CH₂CH₂OH oder (C₁₋₁₂)-Alkyl ist, kann -NRR₁ nicht -NHR* sein, wobei R* Wasserstoff oder (C₁₋₁₀)-Alkyl ist, oder durch Aziridinyl, Pyrrolidyl, Piperidyl und 4-Methylpiperazyl oder 4-Ethylpiperazyl ersetzt sein; (b) wenn R₁ Wasserstoff ist, kann -NR₂R₃ keine Morpholinogruppe sein und (c) die Einheiten -NRR₁ und -NR₂R₃ nicht zwei Morpholinogruppen oder zwei Piperidinogruppen sein können.

2. Verfahren zur Herstellung von Verbindungen der allgemeinen Formel (I) gemäß Anspruch 1, wobei die Verbindungen durch Hydrolyse von Zwischenprodukten der allgemeinen Formel (II) erhalten werden: 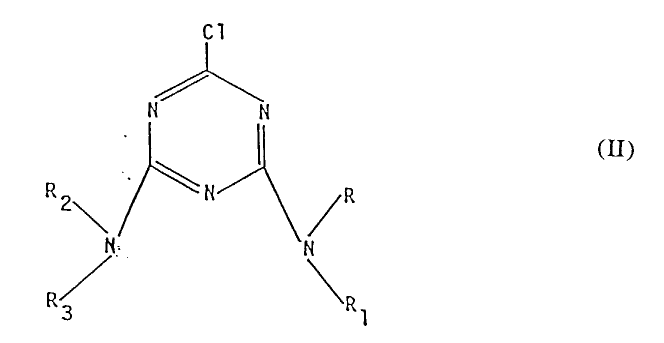 worin R, R₁, R₂ und R₃ die in Anspruch 1 angegebenen Bedeutungen haben.

3. Verfahren gemäß Anspruch 2, wobei die Hydrolysereaktion in Gegenwart einer Säure bei Temperaturen von etwa 60 bis etwa 100°C oder in Gegenwart einer Base bei Temperaturen von etwa 100 bis etwa 180°C durchgeführt wird.

4. Verfahren zur Herstellung von Verbindungen der allgemeinen Formel (I) gemäß Anspruch 1, wobei die Verbindungen durch Hydrolyse von Zwischenprodukten der allgemeinen Formel (Vl) erhalten werden: 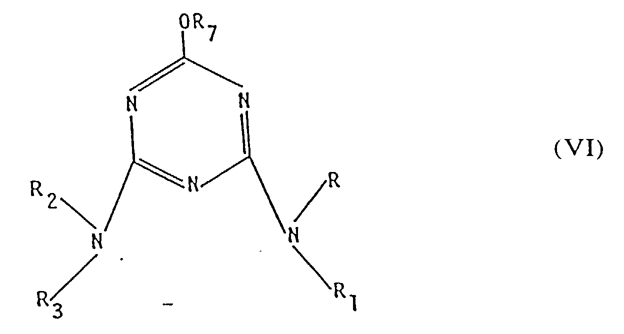 worin R, R₁, R₂ und R₃ die in Anspruch 1 angegebenen Bedeutungen haben und R₇ eine (C₁-C₄)-Alkylgruppe ist.

5. Verfahren gemäß Anspruch 4, wobei die Hydrolysereaktion in Gegenwart einer Säure bei Temperaturen von etwa 80 bis etwa 150°C oder in Gegenwart einer Base bei Temperaturen von etwa 100 bis etwa 180°C durchgeführt wird.

## Revendications

1. Dérivés de l'ameline, de formule générale (I): 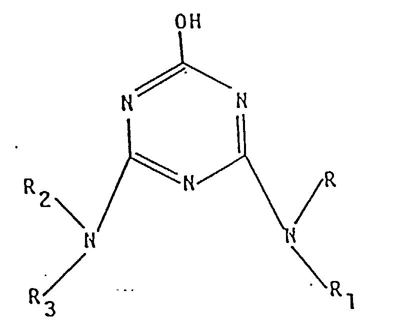 dans laquelle :
R représente un atome d'hydrogène ;
et au moins l'un des groupes symbolisés par R₁, R₂ et R₃ est choisi parmi ceux de formule
―[―CₙH₂ₙ―]―O-R₄
ou 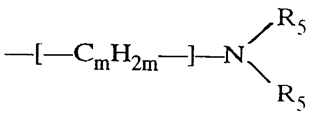
formules dans lesquelles
- n représente un nombre entier qui vaut de 2 à 8,
- m représente un nombre entier qui vaut de 2 à 6,
- R₄ représente
- un atome d'hydrogène,
- un groupe alkyle en C₁₋₈ ou alcényle en C₂₋₆,
- un groupe de formule ―[―CₚH₂ₚ―]―O-R₆ où p représente un nombre entier valant de 1 à 4 et R₆ représente un atome d'hydrogène ou un groupe alkyle en C₁₋₄, ou
- un groupe cycloalkyle en C₆₋₁₂ ou alkyl-cycloalkyle en C₆₋₁₂,
- les groupes représentés par R₅, identiques ou différents l'un de l'autre, sont chacun
- un atome d'hydrogène, ou
- un groupe alkyle en C₁₋₈, alcényle en C₂₋₆, cycloalkyle en C₆₋₁₂, alkyl-cycloalkyle en C₆₋₁₂ ou hydroxyalkyle en C₁₋₄.
- ou le fragment -N(R₅)₂ est remplacé par un groupe hétérocyclique azoté, lié à la chaîne alkyle par l'intermédiaire de l'atome d'azote et choisi parmi les groupes aziridyle, pyrrolidyle, pipéridyle, morpholyle, thiomorpholyle, pipérazyle, 4-méthylpipérazyle et 4-éthylpipérazyle ;
les autres groupes symbolisés par R₁ à R₃, identiques ou différents l'un de l'autre, sont chacun un groupe défini plus haut ou un groupe choisi parmi
- un atome d'hydrogène,
- un groupe alkyle en C₁₋₁₈ ou alcényle en C₂₋₈,
- un groupe cycloalkyle en C₆₋₁₆ ou alkyl-cycloalkyle en C₆₋₁₆, portant éventuellement un substituant hydroxy ou hydroxyalkyle en C₁₋₄ ;
ou bien, dans la formule générale (I), au moins l'un des fragments -NRR₁ et -NR₂R₃ est remplacé par un groupe hétérocyclique azoté, lié au cycle triazine par l'intermédiaire de l'atome d'azote et choisi parmi les groupes aziridyle, pyrrolidyle, pipéridyle, morpholyle, thiomorpholyle, pipérazyle, 4-méthylpipérazyle, 4-éthylpipérazyle, 2-méthylpipérazyle, 2,5-diméthylpipérazyle, 2,3,5,6-tétraméthylpipérazyle, 2,2,5,5-tétraméthylpipérazyle, 2-éthylpipérazyle et 2,5-diéthylpipérazyle ; sous réserve que
a) si l'un des symboles R₂ et R₃ représente un groupe -CH₂CH₂OH et l'autre un groupe -CH₂CH₂OH ou alkyle en C₁₋₁₂, le fragment -NRR₁ ne peut ni être un fragment -NHR* où R* représenterait un atome d'hydrogène ou un groupe alkyle en C₁₋₁₀, ni être remplacé par un groupe aziridyle, pyrrolidyle, pipéridyle, 4-méthylpipérazyle ou 4-éthylpipérazyle ;
b) si R₁ représente un atome d'hydrogène, le fragment -NR₂R₃ ne peut pas être un groupe morpholino, et
c) les fragments -NRR₁ et -NR₂R₃ ne peuvent pas être deux groupes morpholino ou deux groupes pipéridino.

2. Procédé de préparation des composés de formule générale (I) définis dans la revendication 1, dans lequel on obtient ces composés par hydrolyse de composés intermédiaires de formule générale (II) : 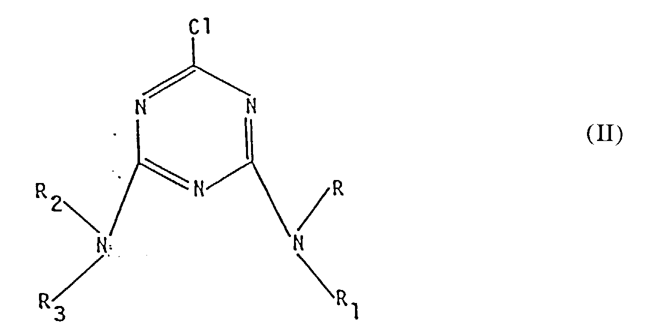 dans laquelle R, R₁, R₂ et R₃ ont les significations indiquées dans la revendication 1.

3. Procédé conforme à la revendication 2, dans lequel on effectue la réaction d'hydrolyse soit en présence d'un acide, à des températures d'environ 60 °C à environ 100 °C, soit en présence d'une base. à des températures d'environ 100 °C à environ 180 °C.

4. Procédé de préparation des composés de formule générale (I) définis dans la revendication 1, dans lequel on obtient ces composés par hydrolyse de composés intermédiaires de formule générale (VI) : 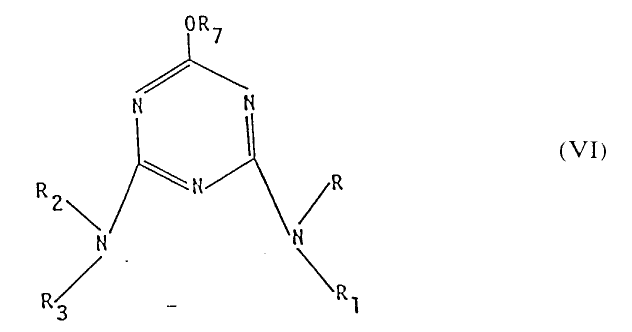 dans laquelle R, R₁, R₂ et R₃ ont les significations indiquées dans la revendication 1, et R₇ représente un groupe alkyle en C₁₋₄.

5. Procédé conforme à la revendication 4, dans lequel on effectue la réaction d'hydrolyse soit en présence d'un acide, à des températures d'environ 80 °C à environ 150 °C, soit en présence d'une base, à des températures d'environ 100 °C à environ 180 °C.
